# EUROPEAN PATENT SPECIFICATION

(11) **EP 3 297 557 B1**
(45) Date of publication and mention of the grant of the patent: **06.11.2019**
(21) Application number: 16725434.1
(22) Date of filing: 17.05.2016
(51) Int. Cl.: A61B 17/80

(54) **A UNIVERSAL PLATE FOR OSTEOSYNTHESIS OF MANDIBULAR CONDYLAR FRACTURE**
UNIVERSELLE PLATTE ZUR OSTEOSYNTHESE EINER MANDIBULÄREN KONDYLENFRAKTUR
PLAQUE UNIVERSELLE POUR OSTÉOSYNTHÈSE DE FRACTURE CONDYLIENNE MANDIBULAIRE

(30) Priority: 18.05.2015 PL 41237515
(43) Date of publication of application: 28.03.2018
(73) Proprietor: Uniwersytet Medyczny w Lodzi, 90-419 Lodz (PL)
(72) Inventor: KOZAKIEWICZ, Marcin, 92-447 Lódz (PL)
(74) Representative: Kaminski, Piotr
(86) International application number: PCT/EP2016/061042
(87) International publication number: WO 2016/184865

(56) References cited:
- WO-A1-2004/045433
- US-A1- 2011 144 698

## Description

The object of the invention is a universal plate for fixing mandibular condylar fracture at each level, to be used both on the left and right side of the patient.

A frequent consequence of injuries is a mandibular condylar fracture resulting in a disfunction in biting, speaking and facial deformation. Temporomandibular joint disorders and malocclusions occur. To restore the efficiency of the joint bone segments should be reduced and fixed rigidly as soon as possible, and also the fixing material should have sufficient strength, because at the time of biting large forces act in this region of the mandible in different directions.

Dedicated plates for fixing condylar fracture in a shape of a delta, triangle, rhombic, letters A, Y, and lambda are known. Some of them are universal (delta-, triangle-, rhombic-shaped plate, in a shape of a letter Y) and some are dedicated to a specific side of the patient (lambda plate and A plate). Some of the plates have small dimensions (triangular plates, rhombic-shaped plates and most delta-shaped plates), which allows for their use with an endoscope assistance. Other plates are relatively large (lambda plate, A, Y plates, some of the delta-shaped plates) and intended for use by conventional skin approach. The advantage of all these dedicated plates is accordance to the lines of compression and stretching of the mandibular condylar bone in the structure, which occur during the act of mastication. This ensures a stable osteosynthesis of fractures and protects against plate breakage during the healing period. Another important design procedure increasing the breaking strength is thickening the bridges present in the lambda plate, triangular plate, and A plate. From the clinical point of view it is important for the upper part of the plate to be narrow, so that it could be screwed into the narrow bone of the neck of the mandible. It is well presented by lambda plate. It was observed that the stability of the fracture fixing depends on the number of fastening screws, in particular in the upper segment. Most dedicated plates have only the possibility of mounting two screws in the upper segment. Stability is much higher when three screws are used, which were applied in plate A. However, a disadvantage of the plate is that the plate is side-dedicated, i.e. different plates are used for the left and right side of the patient.

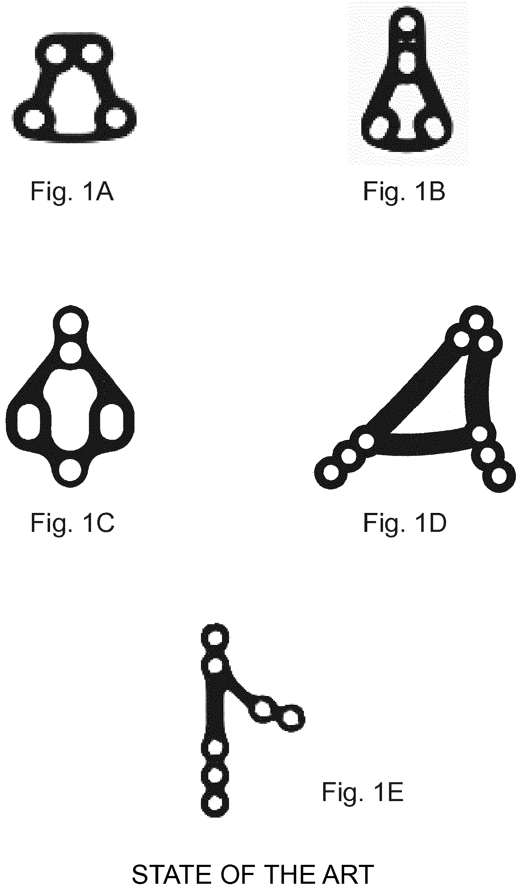

Examples of various types of plates discussed above are shown in Fig. 1A-1E, where Fig. 1A shows an example of a delta plate, Fig. 1B shows an example of a triangle-shaped plate, Fig. 1C shows an example of a rhombic-shaped plate, Fig. 1D shows an example of an A plate and Fig. 1E shows an example of a lambda plate.

Example A plate was discussed in detail in the publication of the Polish patent application no. P.400191. The plate presented therein has two arms connected together at the top and parted at the bottom and there connected with a crosspiece, wherein the screw holes for fixing the plate in the condyle are at the top at the arm joint and, in the bottom of each arm there are holes for screws for fixing the plate in the ramus of the mandible.

WO2004/045433A1 discloses an A-shaped universal plate for fixing mandibular condylar fracture, comprising two arms connected together at the top and parted at the bottom and there connected with a crosspiece. Two holes for screws are provided at the joint of the arms for fixing the plate in the condyle and also two holes for screws for fixing the plate in the ramus of the mandible are provided in the lower and of the arms. The plate is symmetrical relative to an axis of symmetry and the crosspiece has the axis of symmetry coinciding with the axis of symmetry of the plate. Two arms of the crosspiece are directed towards the respective holes for screws fixing the plate in the ramus of the mandible.

US2011/0144698 also discloses a mandibular bone fixation plate configured to be fixed to a mandible, the mandibular bone fixation plate comprising a primary leg; and an auxiliary leg extending obliquely out from the primary leg, such that the primary leg generally corresponds in shape to a posterior border of a mandible, and the auxiliary leg generally corresponds in shape to a sigmoid notch of the mandible.

It would be advisable to develop a new plate structure, which would be bilateral, i.e. universal for the side of the fracture, and would have high strength.

The object of the invention is an universal plate for fixing mandibular condylar fracture, an A-shaped one, comprising two arms connected together at the top and parted at the bottom and there connected with a crosspiece, wherein three holes for screws for fixing the plate in the condyle are located at the joint of said arms, said holes being arranged at the top of the vertices of an isosceles triangle, and at the lower ends of the arms there are two holes for screws for fixing the plate in the ramus of the mandible, the plate being further characterized in that the plate is symmetrical relative to an axis of symmetry and the crosspiece is an X-shaped crosspiece with the axis of symmetry coinciding with the axis of symmetry of the plate, wherein four arms of the crosspiece are directed towards the respective holes for screws for fixing the plate in the ramus of the mandible.

Preferably, the holes for screws for fixing the plate in the condyle are arranged at the vertices of an isosceles triangle with the axis of symmetry coinciding with the axis of symmetry of the plate, wherein the vertex of the triangle opposite the base of the triangle is directed towards the crosspiece.

Preferably, the upper parts of the arms between the holes for screws for fixing the plate in the condyle and the holes for screws for fixing the plate in the ramus of the mandible and the lower parts of the arms between the two holes for screws for fixing the plate in the ramus of the mandible have the shape of curved bridges, bent towards the axis of symmetry of the plate.

Preferably, the width of the upper parts of the arms is greater than the width of the lower parts of the arms.

Preferably, the holes are threaded.

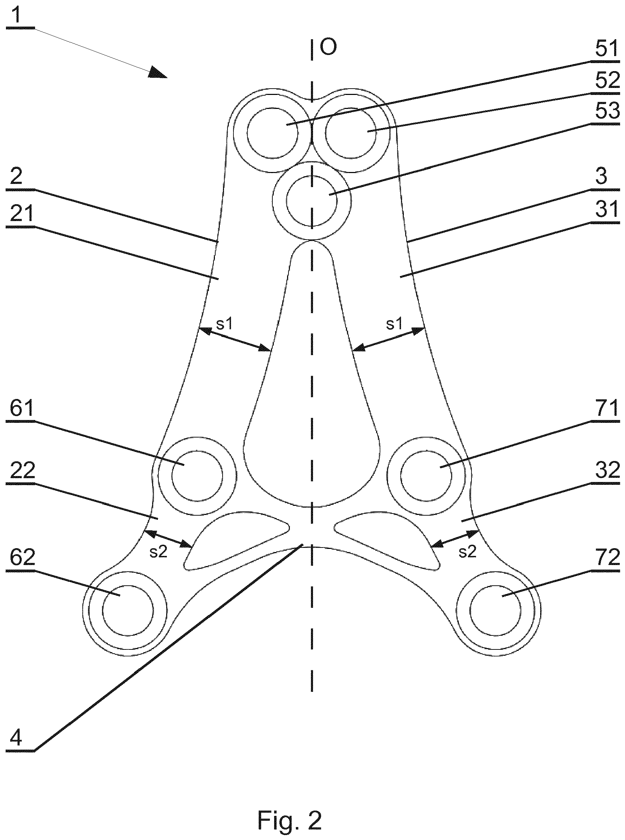

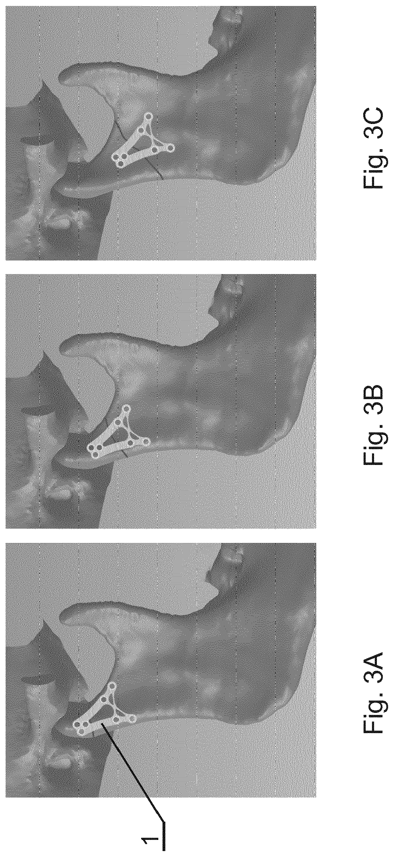

A plate of the invention is shown in the drawing, in which Fig. 2 shows the plate in a front view, Fig. 3A-3C shows a plate on the ramus of the mandible, fixing mandibular condylar fractures at different levels.

A plate 1 of the invention, shown in the embodiment in Fig. 2, has the shape of a symmetrical letter A with the axis of symmetry O, wherein arms 2, 3 are connected with a crosspiece 4. There are three holes 51, 52, 53 at the joint of the arms 2 and 3, and said holes are designed to accommodate screws for fixing the plate 1 in the condyle. The holes 51, 52, 53 are arranged at the vertices of an isosceles triangle having the axis of symmetry coinciding with the axis of symmetry O of the plate, while vertex opposite the base equipped with the hole 53 is directed towards the crosspiece 4. At the lower ends of the arms 2, 3 of the plate 1 there are located pairs of holes 61, 62 i 71, 72 for screws for fixing the plate in the ramus of the mandible (or in the neck of the mandible depending on the level of fixed fracture), wherein one of the holes 61, 71 of each pair is arranged above the crosspiece 4 and the second of the holes 62, 72 of each pair is arranged below the crosspiece 4 (i.e. closer to the end of the arms 2, 3).

The crosspiece 4 is an X-shaped crosspiece with the axis of symmetry coinciding with the axis of symmetry O of the plate, whose four arms are directed towards the respective holes 61, 62 and 71, 72 at the lower ends of the arms 2, 3.

Upper parts 21, 31 of the arms 2, 3 between the holes 51 and 61 and 52 and 71, as well as the lower parts 22, 32 of the arms 2, 3 between the holes 61 and 62 and 71 and 72 have the shape of curved bridges, bent towards the axis of symmetry. A width s1 (measured at the narrowest point) of the upper parts 21, 31 of the arms 2, 3 may be greater, for example by 50% from a width s2 (measured at the narrowest point) of the lower parts 22, 32 of the arms 2, 3.

The plate can be compact, having for example a height of approx. 22 mm and a width of approx. 18 mm and a thickness from 1 mm to 1.3 mm. The width s1 may be 3 mm, and the width s2 may be 2 mm. The holes 51, 52, 53, 61, 62, 71, 72 may be designed to accomodate the screw with a diameter from 1.8 mm to 2.0 mm and they may be threaded.

As shown in Fig. 3A-3C, the plate may be mounted on the ramus of the mandible (where the fracture is marked with a thin line) at different levels. The plate is applied on both sides, i.e. it can be mounted both on the left and right side of the patient. The use of three screw holes for fixing the plate in the condyle provides high stability of the osteosynthetised bone fragments. The design of the plate is determined by the lines of compression and stretching of the mandibular condyle, which also contributes to the stability of the mount. The use of crosspiece 4 increases the rigidity of the plate and prevents from twisting of the plate at its lower part. This allows for reduction of the width of the lower parts of the arms with respect to their upper parts and allows for reduction of the number of screw holes in the lower part of the plate, which reduces the size and weight of the plate.

## Claims

1. A universal plate (1) for fixing mandibular condylar fracture, an A-shaped one, comprising two arms (2,3) connected together at the top and parted at the bottom and there connected with a crosspiece (4), wherein the plate (1) is symmetrical relative to an axis of symmetry (O) and wherein at the lower ends of the arms there are located two holes for screws for fixing the plate in the ramus of the mandible, **characterized in that** three holes (51, 52, 53) arranged at the vertices of an isosceles triangle for screws for fixing the plate in the condyle are located at the top at the arm joint and the crosspiece (4) is an X-shaped crosspiece with the axis of symmetry coinciding with the axis of symmetry (O) of the plate (1), wherein four arms of the crosspiece (4) are directed towards the respective holes (61, 62; 71, 72) for screws for fixing the plate (1) in the ramus of the mandible.

2. The plate according to claim 1, **characterized in that** the holes (51, 52, 53) for screws for fixing the plate (1) in the condyle are arranged at the vertices of an isosceles triangle (5) having the axis of symmetry coinciding with the axis of symmetry (O) of the plate (1), and wherein the vertex of said triangle opposite the base of the triangle is directed towards the crosspiece (4).

3. The plate according to claim 1, **characterized in that** the upper parts (21, 31) of the arms (2, 3) between the holes (51, 52) for screws for fixing the plate in the condyle and the holes (61, 71) for screws for fixing the plate in the ramus of the mandible and the lower parts (22, 32) of the arms (2, 3) between the two holes (61, 62; 71, 72) for screws for fixing the plate in the ramus of the mandible have the shape of curved bridges, bent towards the axis of symmetry (O) of the plate (1).

4. The plate according to claim 3, **characterized in that** the width (s1) of the upper parts (21, 31) of the arms (2, 3) is greater than the width (s2) of the lower parts (22, 32) of the arms (2,3).

5. The plate according to claim 1, **characterized in that** the holes (51, 52, 53, 61, 62, 71, 72) are threaded.

## Patentansprüche

1. Eine Universalplatte (1) zur Fixierung kondylärer Unterkieferfraktur, A-förmig, bestehend aus zwei Armen (2, 3), die miteinander an der Spitze verbundenen und am Unterteil aufgeteilt und dort mit einem Querstück (4) verbunden sind, wobei die Platte (1) gegenüber der Symmetrieachse (O) symmetrisch ist und wobei an den unteren Enden der Arme zwei Löcher für die Schrauben zur Befestigung der Platte am Ramus des Unterkiefers vorhanden sind, **dadurch gekennzeichnet, dass** drei Löcher (51, 52, 53) an den Eckpunkten eines gleichschenkligen Dreiecks für die Schrauben zur Befestigung der Platte am Kondylus sich oben auf der Armverbindung befinden und das Querstück (4) als ein X-förmiges Querstück mit der Symmetrieachse übereinstimmend mit der Symmetrieachse (O) der Platte (1) gestaltet ist, wobei vier Arme des Querstücks (4) auf die jeweiligen Löcher (61, 62, 71, 72) für die Schrauben zur Befestigung der Platte (1) am Ramus des Unterkiefers gerichtet sind.

2. Die Platte nach Anspruch, **dadurch gekennzeichnet, dass** die Löcher (51, 52, 53) für die Schrauben zur Befestigung der Platte (1) am Kondylus an den Eckpunkten eines gleichschenkligen Dreiecks (5) angeordnet sind, über eine mit der Symmetrieachse (O) der Platte (1) übereinstimmende Symmetrieachse verfügen und wobei der Scheitelpunkt des besagten Dreiecks gegenüber der Basis des Dreiecks auf das Querstück (4) gerichtet ist.

3. Die Platte nach Anspruch 1, **dadurch gekennzeichnet, dass** die oberen Teile (21, 31) der Arme (Z. 3) zwischen den Löchern (51, 52) zur Befestigung der Platte am Kondylus und die Löcher für die Schrauben zur Befestigung der Platte am Ramus des Unterkiefers und die unteren Teile (22, 32) der Arme (2, 3) zwischen den beiden Löchern (61, 62, 71, 72) für die Schrauben zur Befestigung der Platte am Ramus des Unterkiefers eine Form von geschwungenen Brücken, gebeugt in Richtung der Symmetrieachse (O) der Platte (1), haben.

4. Die Platte nach Anspruch 3, **dadurch gekennzeichnet, dass** die Breite (s1) der oberen Teile (21, 31) oder Arme (2, 3) größer als die Breite (s2) der unteren Teile (22. 32) der Arme (2, 3) sind.

5. Die Platte nach Anspruch 1, **dadurch gekennzeichnet, dass** die Löcher (51, 52. 53, 61, 62, 71. 72) mit einem Gewinde versehen sind.

## Revendications

1. Une plaque universelle (1) pour fixer une fracture condylienne mandibulaire, en forme de A, comprenant deux bras (2, 3) reliés entre eux en haut et séparés en bas et reliés à une traverse (4), dans laquelle la plaque (1) est symétrique par rapport à un axe de symétrie (O) et dans laquelle, aux extrémités inférieures des bras, il y a deux trous pour vis pour fixer la plaque dans la branche de la mandibule, **caractérisée en ce que** trois trous (51, 52, 53) disposés aux sommets d'un triangle isocèle pour vis pour fixer la plaque dans le condyle sont situés en haut au niveau de l'articulation du bras et que la traverse (4) est une traverse en forme de X avec l'axe de symétrie coïncidant avec l'axe de symétrie (O) de la plaque (1), dans laquelle quatre bras de la traverse (4) sont dirigés vers les trous respectifs (61, 62, 71, 72) pour vis pour fixer la plaque (1) dans la branche de la mandibule.

2. La plaque selon la revendication 1, **caractérisée en ce que** les trous (51, 52, 53) pour vis pour fixer la plaque (1) dans le condyle sont disposées aux sommets d'un triangle isocèle (5) d'axe de symétrie coïncidant avec l'axe de symétrie (O) de la plaque (1), et dans laquelle le sommet de ledit triangle opposé à la base du triangle est dirigé vers la traverse (4).

3. La plaque selon la revendication 1, **caractérisée en ce que** les parties supérieures (21, 31) des bras (2, 3) entre les trous (51, 52) pour vis pour fixer la plaque dans le condyle et les trous (61, 71) pour vis pour fixer la plaque dans la branche de la mandibule et les parties inférieures (22, 32) des bras (2, 3) entre les deux trous (61, 62, 71, 72) pour vis pour fixer la plaque dans la branche de la mandibule ont la forme de ponts incurvés, courbés vers l'axe de symétrie (O) de la plaque (1).

4. La plaque selon la revendication 3, **caractérisée en ce que** la largeur (s1) des parties supérieures (21, 31) ou des bras (2, 3) est supérieure à la largeur (s2) des parties inférieures (22, 32) des bras (2, 3)

5. La plaque selon la revendication 1, **caractérisée en ce que** les trous (51, 52, 53, 61, 62, 71, 72) sont filetés.
